# EUROPEAN PATENT APPLICATION

(11) **EP 4 403 208 A1**
(43) Date of publication of application: **24.07.2024**
(21) Application number: 23152013.1
(22) Date of filing: 17.01.2023
(51) Int. Cl.: A61N 1/36, A61B 5/12, H04R 25/00

(54) **DEVICE FOR DIAGNOSIS AND/ODER SUPPORTING OF HEARING CAPABILITIES**

(71) Applicant: Medizinische Hochschule Hannover, 30625 Hannover (DE)
(72) Inventor: Nogueira Vazquez, Waldo, 30177 Hannover (DE); Büchner, Andreas, 30559 Hannover (DE); Lenarz, Thomas, 30916 Isernhagen (DE); Krüger, Benjamin, 30163 Hannover (DE)
(74) Representative: Gerstein, Hans Joachim

(57) **Abstract**

A device for diagnosis of hearing capabilities of a patient or to support hearing capabilities, comprising a control unit (3), and at least one electrode (1) adapted to simulate auditory nerves of the patient connectable to the control unit (3), wherein the control unit (3) is adapted to electrically stimulate at least one auditory nerve related to the auditory sense of the patient. The device further comprises an acoustic loudspeaker (2) connectable to the control unit (3), wherein the device is adapted to acoustically stimulate at least one of the ear of the patient by emitting acoustic sound with the loudspeaker (2) for acoustic masking, and to electrically stimulate at least one nerve of the patient.

The diagnosis device further comprises at least one sensor element (9) connectable to an auditory unit (10a, 10b, 10c) adapted to measure hearing capabilities of the patient, wherein the device is adapted to determine the hearing capabilities of the patient as a function of acoustic masking and the related electrically stimulation.

## Description

The invention relates to a device for diagnosis of hearing capabilities of a patient, comprising:
- a control unit;
- at least one electrode adapted to simulate auditory nerves of the patient connectable to the control unit, wherein the control unit is adapted to electrically stimulate at least one auditory nerve related to the auditory sense of the patient; and
- at least one sensor element connectable to an auditory unit adapted to measure hearing capabilities of the patient.

The invention further relates to a device for supporting hearing capabilities of a patient comprising:
- a control unit; and
- at least one electrode adapted to stimulate auditory nerves of the patient connectable to the control unit, wherein the control unit is adapted to electrically stimulate at least on auditory nerve related to the auditory sense of the patient.

Hearing loss is the most common sensory deficit in the elderly, and it is becoming a severe social as well as a health problem. Across the whole lifespan, from new-borns to the elderly, hearing loss impairs the exchange of information, thus significantly impacting everyday life, causing loneliness, isolation, dependence, frustration and communication disorders. Cochlear implants are hearing prosthetics that stimulate the auditory nerve with electrodes placed inside the cochlea. Cochlear implants are gradually being implanted in subjects retaining low-frequency residual hearing. In general, these subjects obtain large benefits in speech perception from electric acoustic stimulation, although large variability exists and some subjects do not benefit.

Dorman, M. F., Gifford, R. H., Spahr, A., J., McKarns, S., A. (2008): "The Benefits of Combining Acoustic and Electric Stimulation for the Recognition of Speech, Voice and Melodies", in Audiol Neurotol, 13,105-112 and Imsiecke, M., Büchner, A., Lenarz, T., & Nogueira, W. (2020): "Psychoacoustic and electrophysiological electric-acoustic interaction effects in cochlear implant users with ipsilateral residual hearing", in Hearing Research, 386, 107873. (https://doi.org/10.1016/j.heares.2019.107873) both describes, that for these subjects with residual hearing, combined electric acoustic stimulation significantly improves their speech performance. However, the mechanisms responsible for this benefit have not yet been established.

Krüger, B., Büchner, A., Nogueira, W. (2017): "Simultaneous masking between electric and acoustic stimulation in CI users with residual low-frequency hearing", Hearing Research. 353, 185-196 discovered that electric and acoustic stimulation interact with each other when both stimulation modalities are presented simultaneously.

Imsiecke, I., Krüger, B., Büchner, A., Lenarz, T., Nogueira, W. (2018): "Electric-Acoustic Forward Masking in CI Users with Ipsilateral Residual Hearing", in Hearing Research. 364, 25-37 describes the effect, that interaction refers to suppression or enhancing mechanisms when electric and acoustic stimulation is delivered to the cochlea non-simultaneously one after the other.

Previous approaches investigated some basic mechanisms of electric acoustic interaction through electrophysiological experiments in animals and psychoacoustics in humans.

Vollmer, M., Hartmann, R., and Tillein, J. (2010): "Neuronal responses in cat inferior colliculus to combined acoustic and electric stimulation", in Adv. Otorhinolaryngol. 67, 61-69 showed a reduction in spike activity at the level of the inferior colliculus when an electric masker was simultaneously presented with an acoustic tone suggesting that interaction effects between both modalities occur already at peripheral sites.

Other studies showed that interaction can occur at the level of the cochlea through electric and acoustic excitation of the basilar membrane, the inner hair cells (e.g. Tillein, J., Hartmann, R., Kral, A. (2015): "Electric-acoustic interactions in the hearing cochlea: Single fiber recordings", in Hear Res. 322, 112-26; Sato, M., Baumhoff, P., Kral, A. (2016): "CI stimulation of a hearing ear generates separate electrophonic and electroneural responses", in J. Neurosci. 36, 54-64; Sato, M., Baumhoff, P., Tillein, J., Kral, A. (2017): "Physiological mechanisms in combined electric-acoustic stimulation", in Otol. Neurotol. 38, 215-223) or the outer hair cells (Fräter, A. (2019): "Effects of Electrical Stimulation by Cis on Residual Hearing", in PhD thesis University College London). This is a phenomenon known as electrophony.

Interactions may also occur at the level of the auditory nerve, a phenomenon known as electroneural stimulation (e.g. Stronks, H. C., Versnel, H., Prijs, V. F., de Groot, J. C. M..1., Grolman, W., Klis, S. F. L. (2013): "The Role of Electrophonics in Electroacoustic Stimulation of the Guinea Pig Cochlea", in Qtol. & Neurotology. 34(3), 579-587; Sato, M., Baumhoff, P., Tillein, J., Kral, A. (2017); "Physiological mechanisms in combined electric-acoustic stimulation", in Otol. Neurotol. 38, 215-223).

These previous works showed preliminary interaction effects in animals with very different peripheral physiology than humans and without having the possibility to measure interactions more centrally than the inferior colliculus. For these reasons, the exact interaction mechanisms between electric and acoustic stimulation interaction in humans remain unexplained from the periphery to the central auditory system.

Lin, F. R., Metter, E. J., O'brien, R. J., Resnick, S. M., Zonderman, A. B., & Ferrucci, L. (2011): "Hearing loss and incident dementia", in Archives of neurology. 68, 2, 214-220 were the first to show electric acoustic masking in human cochlear implant users with residual hearing in the same ear in a single subject. This study was limited by the low number of participants, their limited acoustic hearing and the methods which were based only on psychoacoustics.

In the cochlear implant field, low-frequency hearing diagnostic is extremely important and necessary to decide the best treatment by either a hearing aid or a cochlear implant. Moreover in case of cochlear implant treatment, it is required to know the amount of residual hearing to individualize how much or how deep the electrode will be inserted in the cochlea and also to fit or program the cochlear implant and its acoustic component to amplify or not the sound in the low frequencies.

During the cochlear implantation procedure, the residual hearing of the subject is put at risk. Today it is not possible to reliably assess residual hearing during the cochlear implantation putting at risk the residual hearing of the subject.

In general for newborns, babies or people that cannot provide behavioural feedback it is essential to diagnose and characterize hearing loss in the low frequencies. Diagnosis of low frequency hearing loss finds application in indication and fitting of hearing aids.

Providing electric stimulation with a cochlear implant has been shown to be extremely successful.

Diagnostic of hearing loss relies exclusively on physiological or behavioural responses to acoustic only stimulation. Diagnostics of hearing loss in newborns rely on physiological objective estimates of audiometric threshold.

These objective measures include:
1) Otoacoustic emissions (OAEs), acoustic responses generated by the hair cells in the cochlea to acoustic stimuli (e.g. Gong, Q., Liu, Y., & Peng, Z. (2020): "Estimating Hearing Thresholds From Stimulus-Frequency Otoacoustic Emission", in Trends in hearing, 24, 2331216520960053);
2) Electrocochleography (ECochG), neural responses from the hair cells and the auditory nerve elicited by acoustic stimulation (e.g. Gibson, W. P., Moffat, D. A., and Ramsden, R. T. (1977): "Clinical electrocochleography in the diagnosis and management of Meniere's disorder", in Audiology 16, 389-401);
3) Auditory brainstem responses (ABR) elicited by different nuclei in the brainstem evoked by acoustic stimuli (e.g. Valderrama, J. T., de la Torre, A., Alvarez, I., Segura, J. C., Thornton, A. R., Sainz, M., Vargas, J. L. (2014): "Automatic quality assessment and peak identification of auditory brainstem responses with fitted parametric peaks", in Comput Methods Programs Biomed. 114. 3, 262-75);
4) Auditory steady state responses (ASSR), elicited in response to sinusoidal amplitude and/or frequency-modulated tones (e.g. Galambos, R., Makeig, S., Talmachoff, P.J. (1981): "A 40 Hz auditory potential recorded from the human scalp", in Proc Natl Acad Sei. 78: 2643-2647; Gorga, M.P., Kaminski, J.R., Beauchaine, K.A., Jesteadt, W. (1988): "Auditory brainstem responses to tone bursts in normally hearing subjects", in J Speech Hear Res. 31 :87-97).

None of these measurements has been successfully incorporated into the clinic to assess low-frequency hearing because of their inaccuracy below a frequency of about 1 kHz and because some of them are very time consuming to measure. Moreover, these diagnostic methods are limited in their potential for precision diagnostics of individual inner ear hearing loss and the underlying pathophysiology.

Minimally invasive methods can overcome this limitation by intra-cochlear multimodal stimulation of inner ear structures with acoustic and electric stimuli and locally recording the neural responses as shown in animal experiments (e.g Batrel, C., Huet, A., Hasselmann, F., Wang, J., Desmadryl, G., Nouvian, R., et al. (2017): "Mass Potentials Recorded at the Round Window Enable the Detection of Low Spontaneous Rate Fibers in Gerbil Auditory Nerve", in PlosOne. 12. 1, e0169890). Local recording can be obtained using an inner ear probe placed dose to or minimally inserted in the cochlea during a cochlear implant surgery.

In terms of auditory prosthetics based an elecric stimulation of the cochlea several apporaches have been proposed. A natural step to further avoid trauma and at the same time deliver electric stimulation to the cochlea would consist of providing extra-cochlear electric stimulation.

Stevens, S.S., and Jones, R. C. (1939): "The mechanism of hearing by electrical stimulation", in J. Acoust. Soc. Am., 28, 10, 261-269 and Jones, R. C., Stevens, S. S., & Lurie, M. H. (1940): "Three Mechanisms of Hearing by Electrical Stimulation", in The Journal of the Acoustical Society of America, 12(2), 281-290 described the use of alternating current (sinusoidal) electric stimulation to the cochlea.

Three hearing mechanisms were identified.
1) The first was related to the eardrum's conversion of the electric signal into an acoustic signal.
2) The second mechanism was related to the "electromechanical effect," in which electric stimulation caused the hair cells in the cochlea to vibrate, nowadays called electrophonic stimulation.
3) The third mechanism (electroneural mechanism) was related to direct electric activation of the auditory nerve, as the subjects reported noise-like sensation in response to sinusoidal electrical stimulation, much steeper loudness growth with electric currents, and occasionally activation of no auditory facial nerves.

Fourcin, A. J., Douek, E. E., Moore, B. C., Rosen, S., Walliker, J. R., Howard, D. M.; Abberton, E., Frampton, S. (1983): "Speech perception with promontory stimulation", in Ann N Y Acad Sci. 405, 280-94 describes the use of extra-cochlear electrical stimulation through a round window electrode to subjects with profound bilateral hearing loss and showed benefits to support lip-reading for speech understanding.

Wenzel, G. I., Sarnes, P., Warnecke, A., Stöver, T., Jäger, B., Lesinski-Schiedat, A., Lenarz, T. (2015): "Non-penetrating round window electrode stimulation for tinnitus therapy followed by cochlear implantation", in Eur Arch Otorhinolaryngol. 272, 11. 3283-93 describes the use of a non-penetrating single electrode placed in the round window and connected to a cochlear implant speech processor to reduce tinnitus. The device was used to reduce tinnitus and not to transmit speech. Results showed alleviation of tinnitus in some subjects as well as auditory perception through the round window electrode.

Zeng, F. G., Tran, P., Richardson, M. et al. (2019): "Human Sensation of Transcranial Electric Stimulation", in Sci Rep 9, 15247 discloses an optimized configuration using an ear canal electrode and low-frequency sinusoids that delivered maximally approx. 1% of the transcranial current to the auditory nerve. This amount of current was sufficient to produce sound sensation even in deafened ears suggesting that frequency resonances due to neuronal intrinsic electric properties need to be explored for novel brain-computer interfaces. These approaches were conducted with no or very preliminary speech coding strategies. Moreover, these approaches tried to convey the full speech signal with all its fine structure in time, frequency and intensity dimensions through electric stimulation and could not improve the performance of intra-cochlear electric stimulation.

In general, subjects having a low-frequency residual hearing obtain large benefits in speech perception from electric acoustic stimulation by cochlear implants, although large variability exists and some subjects do not benefit. Therefore, it is highly desirable to create objective diagnostics to assess acoustic low-frequency hearing to indicate cochlear implantation, to monitor and preserve hearing during the implantation procedure and to support hearing capabilites of a patient.

It is highly desirable to create objective diagnostic tools to assess low-frequency hearing to indicate cochlear implantation and to monitor low-frequency hearing during the implantation procedure to avoid loss of residual hearing during insertion.

Object of the present invention is to provide a diagnostic device for improved objective diagnosis of residual hearing, preferably in frequencies below 1 kHz, that is clinically applicable, and to provide a respective hearing support device to improve the heraing capabilities.

The object is achieved by the devices comprising the features of claim 1 and 5. Preferred embodiments are disclosed in the depending claims.

The devices comprises an acoustic loudspeaker connectable to the control unit, wherein the device is adapted to acoustically stimulate at least one of the ear of the patient by emitting acoustic sound with the loudspeaker and to electrically stimulate at least one nerve of the patient. The stimulation can be provided simultaneously, in overlapping time sequences or successive, wherein effects of both electrical and acoustical stimulation occur in interacting times, i.e. having a so called simultaneous effect.

The diagnostic device is adapted to determine the hearing capabilities of the patient as a function of acoustic masking and the related electrically stimulation. With the acoustic masking, the perception of one sound is affected by the presence of another sound provided by the acoustic stimulation.

The diagnosis device improves the diagnostics of hearing loss with respect to currently available hearing diagnostics. The device allows for clinical diagnosis of low-frequency hearing loss in subjects that cannot provide a behavioural response (e.g. newborn, babies or subjects with additional disabilities). Further, the device allows monitoring residual hearing during the cochlear implant surgical procedure. The diagnosis device further allows to determine not only the residual hearing, but also the underlying pathophysiology.

While in the prior art, cochlear potentials are investigated by use of electrocochleography (ECochG) during the cochlear implantation procedure as response to acoustic only tones, the present device is based on the finding, that acoustic stimulation leads to acoustic masking. That means that in a subject with residual hearing in particular in the low frequencies (below around 1 kHz) an acoustic tone or signal causes an elevation in the current needed to perceive electric stimulation. For this reason, a subject without residual hearing will not present the threshold elevation required for electric stimulation.

Thus, the acoustic masking is the prerequisite for the effectiveness of electrical stimulation to be able to determine the physiological effect on the electrical stimulation. This novel mechanisms can be therefore used to diagnose low frequency hearing loss using electric stimulation based on an acoustic stimulation. It is therefore proposed to use not only acoustic stimulation but also simultaneous or almost simultaneous electric stimulation during the surgical procedure to monitor residual hearing.

The device makes use of the overlapping effect of the electric responses of acoustic masking and the electric masking caused by electric stimulation.

Moreover, this mechanism can also be used to treat or rehabiltiate hearing loss through a hearing device or auditory prosthetic.

The hearing support device for supporting hearing capabilities further comprises an acoustic loudspeaker connectable to the control unit, wherein the device is adapted to acoustically stimulate at least one of the ear of the patient by emitting acoustic sound with loudspeaker and, simultaneously, to electrically stimulate at least one nerve of the patient.

This treatment device based on both electric and acoustic stimulation improves speech understanding for people exposed to hearing loss by providing acoustic stimulation to their residual hearing and minimally invasive electric stimulation to cover the high frequency hearing loss.

The invention discovered the mechanisms related to electric acoustic stimulation benefits. The ground-breaking nature of this invention is based on the fundamental interaction mechanisms between electric and acoustic stimulation across the auditory pathway, from the cochlea up to the auditory cortex. The fundamental interaction mechanisms are used to design the improved diagnostic device of hearing loss that combine (e.g. minimally invasive) electric stimulation and acoustic stimulation.

The proposed auditory prosthetic makes use of the interaction mechanisms between acoustic and electric stimulation delivered through minimally invasive electrodes.

These devices are beneficial for a large population suffering from hearing loss across the whole lifespan, from young children who will benefit from im proved hearing diagnostics to the elderly population who will benefit from minimally invasive electric acoustic stimulation technology as the treatment for age-related hearing loss.

The diagnostic device for low-frequency hearing combines electric and acoustic stimulation by use of e.g. minimally invasive methods to stimulate and record evoked responses from the inner ear to the cortex. Extra-cochlear electric stimulation is used to modulate electric acoustic interaction and, by use of the device for supporting hearing capabilities, enhance speech understanding in people suffering from hearning loss as a new treatment device for hearing loss.

The at least one sensor element can be adapted to measure voltage responses of the patient, for example compound action potentials, auditory brainstem responses or central auditory evoked potentials. The voltage responses can be measured due to the provision of a minimum threshold current needed to perceive electric stimulation by acoustic masking.

The acoustic stimulation itself does not introduce any electrical interference signals, but merely evokes nerve signals as a reaction of the patient's sensory cells, which are also stimulated by the electrical stimulation. Thus, no electrical interference is caused by the two different stimulations.

A sensor can be adapted for connection to an electrocochleographic (ECochG), evoked compound action potential (eCAP), auditory brainstem response (ABR), auditory stady state response (ASSR), cortical auditory evoked potential (CAEP) or continuous electroencephalography unit. The sensor can be provided on the surface of the head of the patient or introduced into the head skin of the patient. Preferably, non-invasive electrodes or minimally invasive needle electrodes placed on the head of the patient can be used.

The loudspeaker can be provided in form of ear probe adapted for insertion into an ear channel of the patient. An advantage is the reduction of noise effects and the direct sound transmission to the earpiece.

The loudspeaker can also be provided in form of a headset adapted to be placed on the outer ear of the patient. The headset allows the integration of the electrodes used for the electric stimulation in earpads of circum-aural or supra-aural headphones (onear headphones).

At least one electrode of the at least one electrode, which are adapted to stimulate nerves of the patient, can be adapted to be placed on the outer skin of the head of patient to stimulate a nerve arena related to the auditory sense of the patient.

The at least one electrode can be adapted to be placed on the outer skin of the patient. The at least one electrode can be arranged to stimulate the vagus nerve, for example ramus auricularis. The electric stimulation is effective due to the provision of a minimum threshold current provided by the acoustic stimulation, which occurred simultaneously or shortly before and has not yet decayed.

At least one electrode of the at least one electrode, which is adapted to stimulate nerves of the patient, can be adapted to be placed in the inner ear of the patient, in particular in the inner channel and/or in the promontory and/or the around window niche. This allows for electric stimulation of the desired auditory nerve action potential as function pf the selection of position of the at least one stimulation electrode.

The at least one stimulation electrode, which is adapted to electrically stimulate at least one nerve related to the auditory sense of the patient, can be, for example, a ball electrode or needle electrode.

The control unit can be adapted to electrically stimulate at least on nerve of the patient by use of the at least on electrode with unmodulated pulse-trains.

The control unit can be adapted to acoustically stimulate at least one nerve of the patient by use of the at least one acoustic loudspeaker with a signal having a frequency of less then 2 Kilohertz, preferably of 1 Kilohertz and below.

In the following, the invention is exemplarily described by way of embodiments with the enclosed drawings. It shows:
- Figur 1 -: schematic block diagram of a diagnosis device for diagnosis of hearing capabilities;
- Figur 2 -: schematic block diagram of a hearing aid device for supporting hearing capabilities.

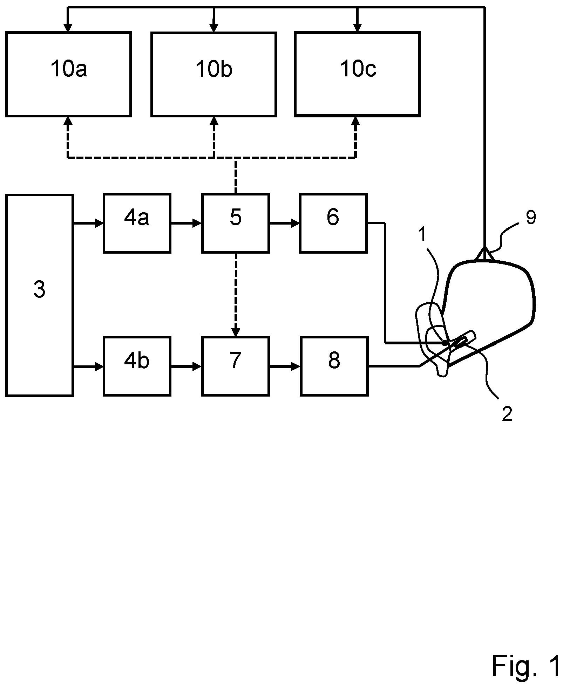

Figur 1 shows a schematic block diagram of a device for diagnosis of hearing capabilities. The diagnostic and monitoring device of hearing loss is based on electric acoustic interaction through electrodes 1 to deliver electric stimulation, e.g. non-invasive, minimally invasive or invasive electrodes 1, and an acoustic loudspeaker 2, e.g. an ear probe or headphone, to deliver acoustic stimulation simultaneously or almost simultaneously to electrically stimulation.

The acoustic stimulation with a sound, preferably a tone of a frequency below 1 kHz, causes an electrical cochlear potential in case that the patient has a residual hearing capability. This electrical effect to the acoustic stimulation, called acoustic masking, provides a minimum threshold current required for the electrical stimulation to be effective. Thus, the acoustical masking is the prerequisite for the electric stimulation and should be provided shortly before the electric stimulation or simultaneously. The time offset between a previous acoustic stimulation and the subsequent electrical stimulation should be chosen so that the effected current has not yet decayed.

The electrical stimulation amplifies the broad effect of acoustic masking, in case that residual hearing capabilities are present.

If the patient's residual hearing is not sufficient, the threshold current will not be reached, so that electrical stimulation will not have a sufficient effect either. The device is therefore arranged to detect the auditory nerve action potentials in response to the acoustical stimulation. The electric stimulus will be masked if residual hearing is available.

In the presence of an acoustic tone the electric stimulus will be masked if residual hearing is available. The electric stimulation can be provided through
a) non-invasive;
b) minimally invasive;
c) invasive electric stimulation.

### a) Non-invasive electric stimulation

Non-invasive electric stimulation will be delivered through an electrode placed in the ear canal. Electric stimulation in the ear canal results in audible sound sensations. For this reason electric stimulation in the ear canal can excite the auditory nerve as a current path exists from the ear canal to the cochlea. The effet of acoustic masking can therefore be measured if an acoustic sound is presented simutaneously or almost simultaneously to ear canal electric stimulation.

### b) Minimally invasive electric stimulation

Minimally invasive electric stimulation can be delivered through a transtympanic needle electrode with the tip of the electrode placed in the promontory. In this way more electric current will end up in the cochlea than with non-invasice electric stimulation. Electric stimulation throguh the needle electrode may provide a more accurate measurement.

### c) Invasive electric stimulation

Invasive electric stimulation can be delivered through an electrode placed in the round window or with an electrode minimally inserted at the base o the cochlea similar to a cochlear implant. This invasive electrode will be inserted prior to cochlear implantation to assess residual hearing with higher precission.

Another option is to deliver electric stimulation invasively through a cochlear implant or through additional electrodes placed in the cochlear implant. This way the electric stimulaton will elicit sound sensation more easily resulting in more precise measures of acoustic masking. However, this procedure will be more invasive and therefore not applicable for subjects that do not undergo a cochlear implant surgery.

The diagnosis device comprises a measurement control unit 3, e.g. a personal computer or controller (µP, µC, FPGA, ASIC etc.) connected via a first galvanic isolation element 4a to a Digital-Analog-Converter 5 providing an analog output signal under control oft he measurement control unit 3. The analog output signal of the Digital-Analog-Converter 5 is connected to an electric stimulation unit 6. The at least one stimulation electrode 1 is connectable to and driven by the electrical signal (e.g. current or voltage signal) provided at the output of the electric stimulation unit 6.

The measurement control unit 3 is further connected via a second galvanic isolation element 4b to a sound generator 7 arranged to provide a sound signal at its output. The sound generator 7 can be designed in form of a frequency generator adapted to provide a periodic signal comprising one specific frequency. In another emodiment, the sound generator 7 can be adapted to provide a sound signal comprising a mix of at least two specific frequencies.

The sound signal can be, for example, a sinus waveform having a frequency below 1 kHz. Optionally, the sound generator 7 can also be adapted to provide a square or triangle form of the periodic signal, or the like.

The sound generator 7 is connected to an audio amplifier 8, wherein the acoustic loudspeaker 2 is electrically connected to the output of the audio amplifier 8.

The cochlear potentials occuring by the acoustic masking and electric stimulation can be measured by use of measurement electrodes 9 placed on the head of the patient and at least one measurement device 10a, 10b, 10b provided to analyse the electrical signals of the measurement electrodes 9.

For example, electrocochleograpy ECochG can be used for recording the electrical potentials generated in the inner ear and auditory nerve in response to the sound stimulation. The measurement device can include an electroencephalograph 10a (EEG) adapted to record an electrogram of the spontaneous electrical activity of the brain.

Alternatively, or in addition, the measurement device can include an otoacoustic emission analyser 10b (OAE) adapted to analyse the otoacoustic emissons based on the electrical signals provided by the measurement electrodes 9.

Alternatively, or in addition, the measurement device can include another type of diagnostic unit 10c adapted to analyse voltage potential evoked by the electric-acoustic masking, e.g. to measure and analyse the auditory brainstem responses (ABR).

The at least one measurement device 10a, 10b, 10c (or measurement unit) is triggered by the measurement control unit 3 or Digital-Analog-Converter 5 in order to synchronise the measurement and analysis of the signals provided by the measurement electrodes 9 with the electric stimulation.

The sound generator 7 is triggered by the measurement control unit 3 or Digital-Analog-Converter 5 in order to synchronise the acoustic stimulation with the electrical stimulation. The acoustic stimulation is triggered before, in overlapping time interval or simultaneously to the electrical stimulation in a way, that acoustic masking is effective at the time of electric stimulation.

The device is therefore adapted to elecit electric acoustic interaction, including acoustic masking. The device is adapted to use of at least one stimulation electrode 1 placed in the ear canal and/or in the promontory and/or the round window niche through transtympanic ball or needle electrodes for cochlear implant preoperative tests. For cochlear implant intraoperative tests needle or ball electrodes can be preferably used. Optionally, additional stimulation electrodes can be embedded in the cochlear implant electrode array.

The diagnostic device is adapted to characterize electric acoustic interaction. In this context, the diagnostic device can be used to assess low-frequency hearing as a way to indicate for hearing aids or cochlear implant and to monitor the status of the low-frequency residual hearing during cochlear implantation as a "cochlear analyzer".

The diagnostic device is adapted to deliver simultaneously or almost simultaneously electric and aocustic stimulation to diagnose hearing loss. The stimulator device is combined with electrophysiological measures to diagnose hearing loss at different stages of the auditory pathway. Electric acoustic interaction will be characterized through electrocochleography, compound action potentials, auditory.brainstem responses, central auditory evoked potentials or any other response to electric and acoustic stimulation.

The interaction between electric and acoustic stimulation is used to elicit the acoustic masking effect through central auditory evoked potentials (CAEPs). A computational model of electric acoustic stimulation may be also incorporated into the device to further improve the diagnosis and monitoring of hearing loss.

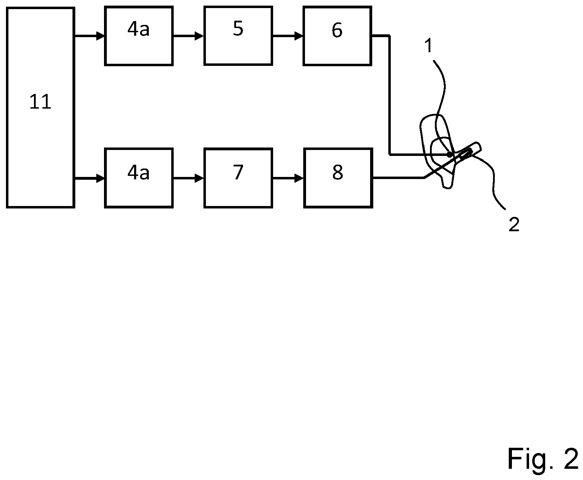

Figur 2 shows a schematic block diagram of a device for supporting hearing capabilities. Similar to the diagnostic device described above, the hearing aid device comprises the means for electric and acoustic stimulation, but without the measurement electrodes 9 and related measurement unit 10a, 10b, 10c.

The hearing support device comprises a control unit 11, e.g. programmed data processor, controller or signal processor (µP, µC, FPGA, ASIC etc.) connected via a first galvanic isolation element 4a to a Digital-Analog-Converter 5 providing an analog output signal under control oft he measurement control unit 3. The analog output signal of the Digital-Analog-Converter 5 is connected to an electric stimulation unit 6. The at least one stimulation electrode 1 is connectable to and driven by the electrical signal (e.g. current or voltage signal) provided at the output of the electric stimulation unit 6.

The control unit 11 of the hearing support device is further connected via a second galvanic isolation element 4b to a sound generator 7 arranged to provide a sound signal at ist output. The sound generator 7 my comprise a frequency generator adapted to provide a periodic signal comprising one specific frequency. Preferably, the sound generator 7 can be adapted to provide a mix of a low frequency used for acoustic stimulation and an amplified sound signal provided from a microphone connected to the hearing support device to receive and amplify sound of the surrounding of the patient in a frequency range above the low-frequency range used for acoustic stimulation. Thus, the acoustic signal emitted by the acoustic loudspeaker 2 can be a mix of the acoustic stimulation frequency and the microphone output signal, i.e. the amplified sound of the surrounding of the patient.

The acoustic stimulation signal can be, for example, a sinus waveform having a frequency below 1 kHz. Optionally, the sound generator 7 can also be adapted to provide a square or triangle form of the periodic signal, or the like for the acoustic stimulation signal.

The sound generator 7 is connected to an audio amplifier 8, wherein the acoustic loudspeaker 2 is electrically connected to the output of the audio amplifier 8.

The device for supporting hearing capabilities of a patient can be an auditory prosthetic or hearing device which provides extra-cochlear electric stimulation and simultaneous acoustic stimulation. The device uses electric acoustic interaction as a means of leveraging the stimulation delivered both electrically and acoustically.

The prosthetic or hearing device uses electric stimulation delivered non-invasively, or minimally invasively. For example, an ear canal electrode or a needle electrode can be provided for electric stimulation.

Electric stimualtion is used to transmit high frequencies and acoustic stimulation is used to transmit low frequencies. The acoustic masking mechanism is used to modulate and leverage the low frequency and high frequency content to minimize the interaction between both modalities.

For example, low-frequency is below 1 kHz and high frequency is above 1 kHz.

There are at least three options to implement the hearing aid.

The first is the use of the diagnostic device as a disgnostic tool. The results can be used as input parameters for characterizing a signal processor of a conventional hearing aid.

The second option is to implement the diagnostic unit in a hearing aid, wherein the device is designed to frequently adapt the setting by diagnosted interaction mechanisms from acoustical and electrical stimulation.

The third option is the use of the interaction mechanisms in real time to amplify or dampen noise or sharpen the audible hearing signal.

## Claims

1. Device for diagnosis of hearing capabilities of a patient, comprising:
- a control unit (3);
- at least one electrode (1) adapted to simulate auditory nerves of the patient connectable to the control unit (3), wherein the control unit (3) is adapted to electrically stimulate at least one auditory nerve related to the auditory sense of the patient; and
- at least one sensor element (9) connectable to an auditory unit (10a, 10b, 10c) adapted to measure hearing capabilities of the patient,
**characterized in, that**
the device further comprises an acoustic loudspeaker (2) connectable to the control unit (3), wherein the device is adapted to acoustically stimulate at least one of the ear of the patient by emitting acoustic sound with the loudspeaker (2) for acoustic masking, and to electrically stimulate at least one nerve of the patient, wherein the device is adapted to determine the hearing capabilities of the patient as a function of acoustic masking and the related electrically stimulation.

2. Device according to claim 1, **characterized in that** the at least one sensor element (9) is adapted to measure voltage responses of the patient.

3. Device according to claim 2, **characterized in that** the at least one sensor element (9) is adapted to measure compound action potentials, auditory brainstem responses or central auditory evoked potentials.

4. Device according to claim 2 or 3, **characterized in that** a sensor element (9) is adapted for connection to an electrocochleographic unit.

5. Device for supporting hearing capabilities of a patient comprising:
- a control unit (11);
- at least one electrode (1) adapted to stimulate nerves of the patient contactable to the control unit (11), wherein the control unit (11) is adapted to electrically stimulate at least on nerve related to the auditory sense of the patient,
**characterized in, that**
the device further comprises an acoustic loudspeaker (2) connectable to the control unit (11), wherein the device is adapted to acoustically stimulate at least one of the ear of the patient by emitting acoustic sound with the loudspeaker (2) for acoustic masking, and to electrically stimulate at least one nerve of the patient based on the acoustic masking.

6. Device according to one of the claims 1 to 5, **characterized in, that** the loudspeaker (2) is provided in form of ear probe adapted for insertion into an ear channel of the patient.

7. Device according to one of claims 1 to 5, **characterized in, that** the loudspeaker (2) is provided in form of headset adapted to be placed on the outer ear of the patient.

8. Device according to one of the preceding claims, **characterized in, that** at least one electrode (1) of the at least one electrode (1) adapted to stimulate nerves of the patient is adapted to be placed on the outer skin of the head of patient to stimulate a nerve arena related to the auditory sense of the patient.

9. Device according to claim 8, **characterized in, that** the at least one electrode (1) adapted to be placed on the outer skin is provided to stimulate the vagus nerve, for example ramus auricularis.

10. Device according to one of the preceding claims, **characterized in**, at least one electrode (1) of the at least one electrode (1) adapted to stimulate nerves of the patient is adapted to be placed in the inner ear of the patient, in particular in the inner channel and/or in the promontory and/or the around window niche.

11. Device according to one of the preceding claims, **characterized in, that** the at least one electrode (1) adapted to electrically stimulate at least one nerve related to the auditory sense of the patient is a ball electrode or needle electrode.

12. Device according to one of the preceding claims, **characterized in, that** the control unit (3, 11) is adapted to electrically stimulate at least on nerve of the patient by use of the at least on electrode (1) with unmodulated pulse-trains.

13. Device according to one of the proceeding claims, **characterized in, that** the control unit (3, 11) is adapted to acoustically stimulate at least one nerve of the patient by use of the at least one acoustic loudspeaker (2) with a signal having a frequency of less than 2 Kilohertz, preferably of 1 Kilohertz and below.

14. Device according to one of the proceeding claims, **characterized in, that** the device is adapted to simultaneously stimulate the auditory nerves by acoustic stimulation and electrically stimulation.
